# FASCICULE DE BREVET EUROPEEN

(11) **EP 2 569 623 B1**
(45) Date de publication et mention de la délivrance du brevet: **12.03.2014**
(21) Numéro de dépôt: 11718721.1
(22) Date de dépôt: 05.05.2011
(51) Int. Cl.: G01N 33/18, G01N 1/16

(54) **SYSTEME DE MESURE OU DE SURVEILLANCE DE LA QUALITE D'UN MILIEU LIQUIDE A FAIBLE CONSOMMATION D'ENERGIE**
SYSTEM ZUR MESSUNG ODER ÜBERWACHUNG DER QUALITÄT EINES FLÜSSIGMEDIUMS MIT GERINGEM ENERGIEVERBRAUCH
SYSTEM FOR MEASURING OR MONITORING THE QUALITY OF A LIQUID MEDIUM WITH LOW ENERGY CONSUMPTION

(30) Priorité: 10.05.2010 FR 1053622
(43) Date de publication de la demande: 20.03.2013
(73) Titulaire: Institut Français de Recherche pour l'Exploitation de la Mer (IFREMER), 92138 Issy-les-Moulineaux Cedex (FR)
(72) Inventeur: JEGOU, Paul, F-29200 Brest (FR); LE PIVER, David, F-29200 Brest (FR); PODEUR, Christian, F-29200 Brest (FR); WOERTHER, Patrice, F-29280 Plouzane (FR)
(74) Mandataire: Novagraaf Technologies
(86) Numéro de dépôt international: PCT/EP2011/057179
(87) Numéro de publication internationale: WO 2011/141343

(56) Documents cités:
- FR-A1- 2 461 254
- FR-A1- 2 741 446
- US-A- 5 633 460

## Description

La présente invention concerne, de façon générale, la surveillance de la qualité d'un milieu liquide, et en particulier des eaux plus ou moins salées dans les zones côtières ou les estuaires.

Plus précisément, l'invention concerne un système de mesure ou de surveillance de la qualité d'un milieu liquide comprenant des premier et second points de prélèvement d'échantillon dans le milieu liquide et un circuit hydraulique dans lequel circulent les échantillons prélevés à partir desdits premier et second points de prélèvement d'échantillon. Le circuit hydraulique comprend notamment
- une chambre de mesure dotée d'au moins un capteur de mesure apte à mesurer une grandeur caractéristique du milieu liquide, tel qu'un paramètre physico-chimique ou biologique du milieu liquide, ladite chambre de mesure présentant une première ouverture en communication fluidique avec le premier point de prélèvement d'échantillon et une seconde ouverture, et
- une première pompe aspirante présentant une entrée d'aspiration reliée à la seconde ouverture de la chambre de mesure et une sortie de refoulement en communication fluidique avec le milieu liquide, la première pompe aspirante étant apte en configuration de fonctionnement à faire circuler dans le circuit hydraulique un échantillon prélevé à partir du premier point de prélèvement d'échantillon.

Un système de mesure ou de surveillance de ce type est par exemple décrit dans la demande de brevet FR 2 741 446.

Pour fonctionner correctement, ce système est complété par un désaérateur placé au-dessus de la première pompe pour piéger les gaz présents dans le circuit lors de la mise en fonctionnement du système ainsi que les gaz issus du dégazage naturel du liquide prélevé. Il comprend également une pompe d'amorçage raccordée au désaérateur pour amorcer le circuit hydraulique en début de fonctionnement et un système de prélèvement d'échantillon pour raccorder sélectivement les points de prélèvement d'échantillons à l'entrée et à la sortie du circuit hydraulique.

Bien que donnant toute satisfaction sur le plan de ses performances, ce système de mesure présente quelques caractéristiques perfectibles, tels qu'une complexité structurelle et une consommation énergétique relativement élevées.

Dans ce contexte, la présente invention a pour but de proposer un système de mesure ou de surveillance d'un milieu liquide présentant une consommation d'énergie réduite ainsi qu'un faible encombrement, et offrant une grande facilité de maintenance.

A cette fin, le système de mesure de l'invention, qui est par ailleurs conforme à la définition générique qu'en donne le préambule ci-dessus, est essentiellement caractérisé en ce que le circuit hydraulique comporte en outre
- une deuxième pompe aspirante présentant une entrée d'aspiration reliée à la première ouverture de la chambre de mesure et une sortie de refoulement en communication fluidique avec le milieu liquide, ladite deuxième pompe aspirante étant apte en configuration de fonctionnement à faire circuler dans le circuit hydraulique un échantillon prélevé à partir du deuxième point de prélèvement d'échantillon;
- au moins une conduite pour raccorder la première ouverture de la chambre de mesure à l'entrée d'aspiration de la deuxième pompe aspirante ou raccorder la deuxième ouverture de la chambre de mesure à l'entrée d'aspiration de la première pompe aspirante, ladite conduite étant munie d'un dispositif de purge disposé en un point haut du circuit hydraulique pour évacuer les gaz présents dans le circuit hydraulique,
et en ce que la sortie de refoulement de la première pompe aspirante est raccordée au deuxième point de prélèvement d'échantillon et la sortie de refoulement de la deuxième pompe aspirante est raccordée au premier point de prélèvement d'échantillon, lesdites première et deuxième pompes aspirantes étant, à l'arrêt, aptes à autoriser le passage de liquide de leur sortie de refoulement vers leur entrée d'aspiration.

Ainsi, selon l'invention, le système de surveillance comprend deux pompes aspirantes disposées de part et d'autre de la chambre de mesure. Grâce à cet agencement, les échantillons de liquide prélevés aux premier et second points de prélèvement circulent dans des sens opposés dans la chambre de mesure et tous les éléments du circuit hydraulique dans lesquels circulent les échantillons de liquide sont disposés en ligne (pas d'élément monté en dérivation). Il en résulte que la perte de charge subie par le liquide en mouvement dans le circuit hydraulique est faible, ce qui permet d'utiliser des pompes aspirantes ayant une puissance nominale réduite. Cet agencement en ligne contribue également à réduire le dépôt dans le circuit hydraulique d'éléments solides tels que des sédiments.

Pour faciliter la maintenance du système et le protéger contre les bio-salissures, le circuit hydraulique comprend en outre avantageusement un moyen de génération ou d'injection de produit biocide disposé entre lesdits premier et deuxième points de prélèvement. Le produit biocide à utiliser est déterminé en fonction du milieu liquide à traiter. Il s'agit par exemple de chlore lorsque le système est destiné à être utilisé en eau salée. L'agencement en ligne de tous les éléments du circuit permet alors, par la simple circulation du liquide dans le circuit, de protéger l'ensemble de ces éléments et de disposer le point de génération ou d'injection indifféremment sur la ligne. Toutefois, pour des raisons d'alternance du sens de circulation et de proximité des capteurs à protéger, ce point d'injection ou de génération est avantageusement monté sur la chambre de mesure.

Avantageusement, les première et seconde pompes aspirantes sont adaptées à une immersion et sont, en configuration de fonctionnement, immergées dans le milieu liquide.

Selon un mode de réalisation préféré, le dispositif de purge est également adapté à une immersion et est immergé dans le milieu liquide. Dans ce mode de réalisation, le dispositif de purge étant positionné en un point haut du circuit hydraulique, le circuit hydraulique est entièrement immergé dans le milieu liquide.

Selon un mode de réalisation particulier, le dispositif de purge comprend avantageusement un système de clapet pour évacuer les gaz présents dans le circuit hydraulique.

Selon un mode de réalisation particulier, le dispositif de purge comprend:
- un corps tubulaire sensiblement vertical monté par son extrémité ouverte inférieure sur une ouverture supérieure de la conduite entre la chambre de mesure et la première ou deuxième pompe aspirante, et
- un clapet cylindrique apte à coulisser dans le passage interne du corps tubulaire et à coopérer avec une portion inférieure du corps tubulaire, formant siège, pour obturer l'extrémité ouverte inférieure dudit corps tubulaire.

Selon un mode de réalisation particulier, la paroi intérieure de la portion inférieure du corps tubulaire comprend une portion tronconique formant le siège du clapet. Dans ce mode de réalisation, le clapet comporte avantageusement une portion inférieure dont la paroi extérieure comprend une portion tronconique coopérant avec la portion tronconique de la paroi intérieure du corps tubulaire pour obturer l'extrémité ouverte inférieure du corps tubulaire.

Le corps tubulaire et le clapet du dispositif de purge automatique sont de préférence en alliage cuivreux anti-bio salissure.

D'autres caractéristiques et avantages de l'invention apparaîtront plus clairement au cours de la description explicative détaillée qui va suivre, en se référant ci-dessous aux dessins annexes, lesquels représentent:
- la figure 1, le schéma de principe d'un système de mesure et de surveillance conforme à l'invention;
- la figure 2, une vue externe en perspective du dispositif de purge utilisé dans le système de mesure et de surveillance de l'invention, ledit dispositif comprenant un corps tubulaire et un clapet apte à coulisser dans le passage interne du corps tubulaire;
- la figure 3, une vue de dessus du corps tubulaire du dispositif de purge de la figure 2;
- la figure 4, une vue en coupe du corps tubulaire, prise selon la ligne IV-IV de la figure 3;
- la figure 5, une vue de dessous du clapet du dispositif de purge de la figure 2; et
- la figure 6, une vue en coupe du clapet, prise selon la ligne VI-VI de la figure 5.

Comme indiqué précédemment, l'invention concerne un système de mesure ou de surveillance d'un milieu liquide, notamment destiné à détecter d'éventuelles pollutions dans les eaux salées ou non, à permettre la modélisation d'écosystèmes, ou encore à permettre de contrôler certaines opérations liées à l'aquaculture, telles que l'irrigation des claires d'affinage d'huîtres par exemple.

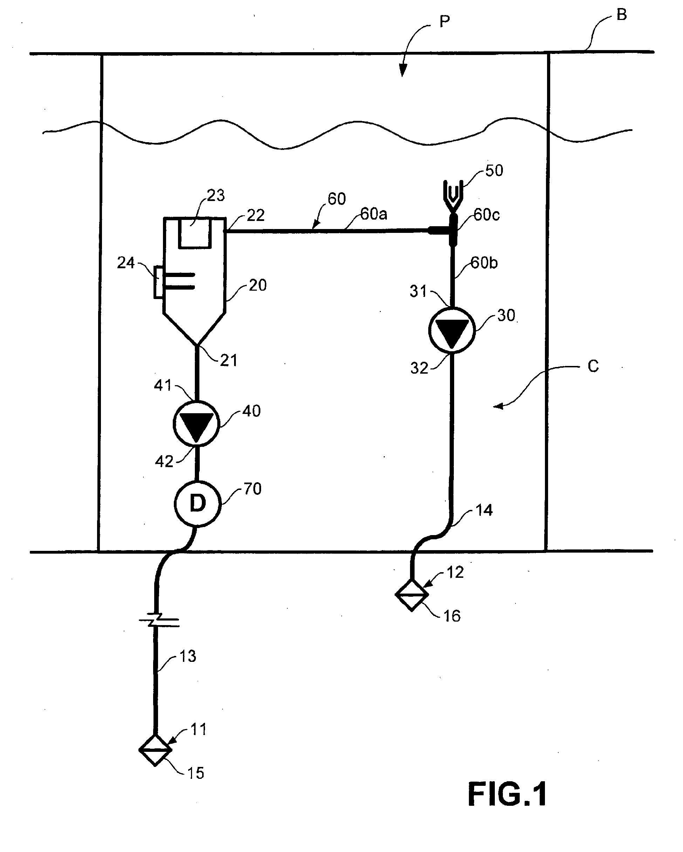

En référence à la figure 1, le système de mesure comprend essentiellement un circuit hydraulique C monté entre deux points de prélèvement d'échantillon 11 et 12. Ce système est destiné à être immergé entièrement dans le milieu liquide. Il est implanté dans le puits P d'une bouée B ou de tout autre support.

Les deux points de prélèvement d'échantillon 11 et 12, qui correspondent aux orifices d'extrémité inférieure de conduites de prélèvement 13 et 14, sont de préférence situés à des profondeurs différentes. Ces points de prélèvement sont avantageusement équipés de crépines, respectivement 15 et 16, pour filtrer le liquide admis dans les conduites de prélèvement 13 et 14.

Le circuit hydraulique C comprend essentiellement une chambre de mesure 20, deux pompes 30 et 40 aspirantes pour prélever des échantillons de liquide aux points de prélèvement d'échantillon 11 et 12, et un dispositif de purge 60 pour évacuer le gaz présent dans le circuit hydraulique C.

La chambre de mesure 20 est dotée d'un ou plusieurs capteurs de mesure, se présentant par exemple sous la forme d'une sonde de mesure multi-paramètres 23, pour mesurer une ou plusieurs grandeurs caractéristiques du milieu liquide à caractériser. Ces grandeurs sont par exemple des paramètres physico-chimiques ou biologiques du milieu liquide. Ces paramètres sont par exemple la température du liquide, sa conductivité électrique, sa turbidité, son pH, sa concentration en oxygène dissous, et/ou sa concentration en chlorophylle.

La chambre de mesure 20, qui est de préférence disposée en configuration sensiblement verticale, présente deux ouvertures, l'une 21 située dans sa partie inférieure et l'autre 22 dans sa partie supérieure.

Les pompes 30 et 40 sont destinées à amener dans la chambre de mesure 20 des échantillons prélevés respectivement aux points de prélèvement d'échantillon 11 et 12.

Les pompes 30 et 40 présentent chacune une entrée d'aspiration, respectivement 31 et 41, et une sortie de refoulement, respectivement 32 et 42.

L'entrée d'aspiration 31 de la pompe 30 est reliée à l'ouverture 22 de la chambre de mesure via une conduite 60 et la sortie de refoulement 32 de la pompe 30 est raccordée au point de prélèvement d'échantillon 12 via la conduite de prélèvement 14. Le dispositif de purge automatique 50 est monté sur la conduite 60.

De même, l'entrée d'aspiration 41 de la pompe 40 est reliée à l'ouverture 21 de la chambre de mesure 20 et la sortie de refoulement 42 est raccordée au point de prélèvement d'échantillon 11 via la conduite de prélèvement 13.

Cette disposition des pompes implique que les échantillons prélevés au point 11 et les échantillons prélevés au point 12 circulent dans des sens opposés dans le circuit hydraulique.

Les pompes sont avantageusement immergées dans le milieu liquide et fonctionnent en alternance, c'est-à-dire que lorsque l'une des pompes est en fonctionnement, l'autre est à l'arrêt et inversement. Entre deux phases de fonctionnement, les deux pompes sont à l'arrêt, notamment pour évacuer les gaz présents dans le circuit hydraulique.

Selon une caractéristique importante de l'invention, les pompes 30 et 40 sont telles que, à l'arrêt, elles autorisent le passage de liquide de leur sortie de refoulement vers leur entrée d'aspiration. Ce sont par exemple des pompes centrifuges à rouet.

Ainsi, avec ce mode de fonctionnement et ce type de pompe, le liquide prélevé au point de prélèvement d'échantillon 11 est refoulé, après passage dans le circuit hydraulique, au point de prélèvement d'échantillon 12 et inversement.

Plus précisément, lorsque la pompe 30 est en fonctionnement, elle aspire du liquide prélevé au point de prélèvement d'échantillon 11. Ce liquide traverse la pompe 40 à l'arrêt avec une perte de charge réduite pour atteindre la chambre de mesure 20 puis traverse la pompe 30 pour être refoulé dans le milieu liquide au point de prélèvement d'échantillon 12. De même, lorsque la pompe 40 est en fonctionnement, elle aspire du liquide prélevé au point de prélèvement d'échantillon 12. Ce liquide traverse la pompe 30 à l'arrêt avec une perte de charge réduite pour atteindre la chambre de mesure 20 puis traverse la pompe 40 pour être refoulé dans le milieu liquide au point de prélèvement d'échantillon 11.

Le brassage produit par la pompe en fonctionnement ne perturbe pas les mesures dans la chambre de mesure 20 car cette pompe est toujours celle qui est disposée en aval de la chambre de mesure.

Le système de mesure de l'invention comprend avantageusement un moyen générateur de produit biocide 24 disposé entre les deux points de prélèvement 11 et 12. Ce moyen générateur de produit biocide est de préférence monté sur la chambre de mesure 20. Lorsque le système est destiné à être utilisé en eau salée, ce moyen 24 est par exemple un moyen générateur de chlore.

Le circuit hydraulique étant un circuit en ligne, un débitmètre peut être interposé en n'importe quel point du circuit. Dans l'exemple de la figure 1, un débitmètre 70 est monté sur la sortie de refoulement de la pompe 40.

Le circuit hydraulique comprend également un dispositif de purge 50 pour évacuer les gaz présents dans le circuit hydraulique, notamment les gaz présents lors de la mise en fonctionnement dudit circuit, les gaz issus du dégazage naturel du liquide dans le circuit et les gaz produits par le dispositif générateur de produit biocide. Ce dispositif est positionné à un point haut du circuit hydraulique, entre les deux pompes 30 et 40.

Dans la forme de réalisation présentée à la figure 1, le dispositif de purge 50 est monté sur la conduite 60. Dans cet exemple, la conduite 60 comprend une portion 60a tubulaire horizontale et une portion 60b tubulaire verticale reliées entre elles par une portion 60c tubulaire en forme de T pivoté de 90° dans le sens horaire. Plus précisément, l'une des extrémités de la portion 60a horizontale est raccordée à l'ouverture 22 de la chambre de mesure et l'autre extrémité est raccordée à la jambe du T. L'extrémité supérieure de la portion 60b verticale est raccordée à un bras du T, dit bras inférieur, et l'extrémité inférieure est raccordée à l'entrée d'aspiration 31 de la pompe 30. Enfin, le dispositif de purge 50 est monté sur l'autre bras du T, dit bras supérieur.

Selon un mode de réalisation préféré illustré par les figures 1 à 6, le dispositif de purge automatique 50 est un système de clapet.

Ce système à clapet comprend un corps 51 globalement tubulaire présentant un passage 510 interne, une extrémité 511 supérieure ouverte et une extrémité 512 inférieure ouverte, et un clapet 52 globalement cylindrique apte à coulisser dans le passage interne 510 du corps tubulaire.

Plus précisément, le passage interne du corps tubulaire comprend, de l'extrémité 511 supérieure à l'extrémité 512 inférieure, une partie 513 supérieure sensiblement cylindrique, et une partie 514 inférieure comprenant une portion 514a tronconique, qui converge en direction de l'orifice situé à l'extrémité 512 inférieure et qui définit un siège d'obturation pour le clapet 52, se prolongeant par une portion 514b finale sensiblement cylindrique.

Le corps tubulaire 51 est par ailleurs muni, à proximité de son extrémité 512 inférieure, d'une collerette 515 d'assemblage pour son assemblage, par vissage par exemple, au bras supérieur de la portion 60c en T. Il comprend également des fentes longitudinales 516a et 516b diamétralement opposées, ménagées dans la portion cylindrique 513. Ces fentes longitudinales peuvent s'étendre jusqu'au bord annulaire de l'extrémité supérieure 511.

Le corps tubulaire 51 comprend également des ouvertures 517a et 517b diamétralement opposées, ménagées dans la portion cylindrique 513 à proximité du bord annulaire de l'extrémité supérieure 511. Ces ouvertures sont destinées à recevoir une goupille (non représentée) destinée à empêcher le clapet 52 de sortir du passage interne 510 du corps tubulaire.

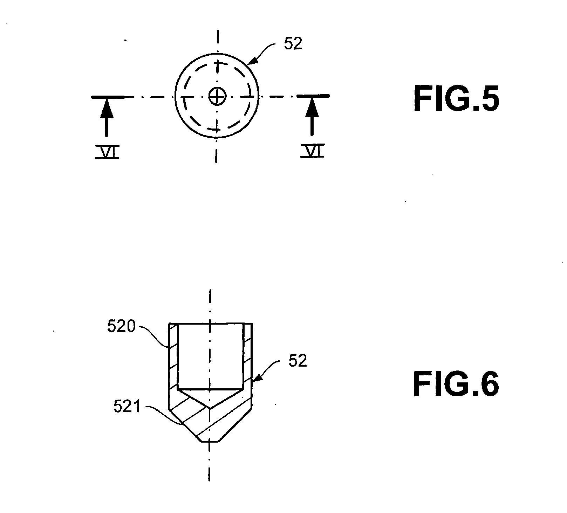

En référence aux figures 5 et 6, le clapet 52 est formé d'une portion 520 supérieure globalement cylindrique se prolongeant par une portion 521 inférieure de forme tronconique.

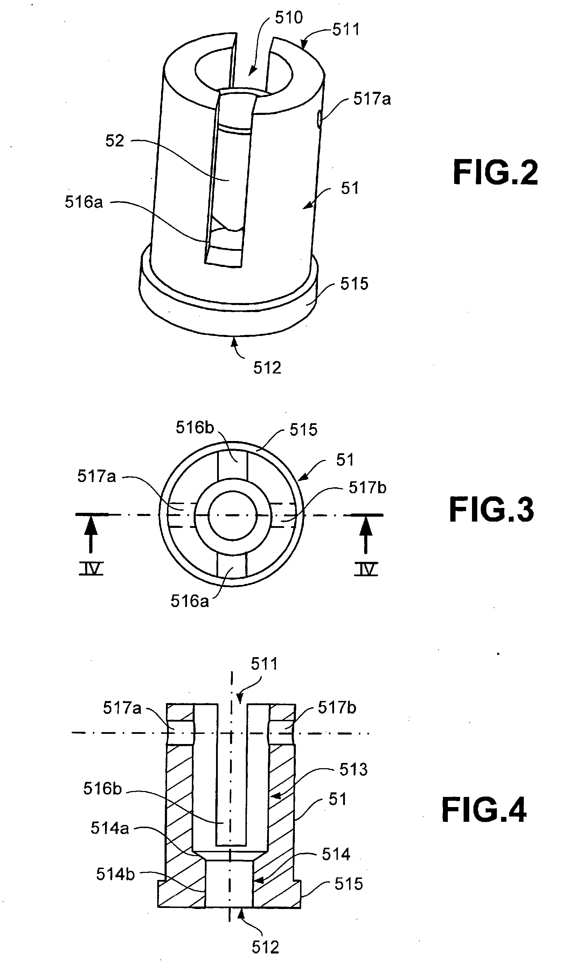

En l'absence de gaz dans le circuit hydraulique et lorsque les pompes 30 et 40 sont à l'arrêt, la portion 521 tronconique du clapet 52 vient en appui par gravité sur la portion 514a tronconique et obture l'orifice de l'extrémité 512 inférieure du corps tubulaire. Le poids du clapet 52 est défini pour que, en présence de gaz dans la conduite 60, le clapet 52 se soulève et que ce gaz s'échappe à l'extérieur du circuit hydraulique en passant par la portion 514 et les fentes longitudinales 517a et 517b du corps tubulaire. Le poids du clapet est par exemple défini pour s'ouvrir sous l'effet d'une bulle de gaz de 1 cm de hauteur sous le clapet. Dans l'exemple des figures 2, 5 et 6, le clapet 52 est creux (la portion 520 est tubulaire).

Lorsque les pompes 30 et 40 sont à l'arrêt, les bulles de gaz s'évacuent en soulevant le clapet 52. Une fois les bulles évacuées, le clapet 52 revient en appui sur le corps 51 pour assurer l'étanchéité entre l'intérieur du circuit hydraulique et le milieu liquide. Ce dispositif de purge est continu et automatique en ce sens qu'il se met en route dès qu'un volume donné de gaz est présent dans la conduite 60.

Lorsque l'une des pompes est en fonctionnement, l'effet de dépression créé par celle-ci s'ajoute au poids du clapet 52 pour plaquer celui-ci contre le corps 51 et augmenter l'étanchéité du dispositif de purge.

L'étanchéité du dispositif de purge est telle que, lorsque l'on prélève du liquide au point 11 (circulation des échantillons du point 11 vers le point 12), l'aspiration soit suffisante dans la chambre de mesure et, lorsque l'on prélève du liquide au point 12 (circulation des échantillons du point 12 vers le point 11), la quantité de liquide provenant d'une éventuelle fuite du dispositif de purge soit faible. Dans ce dernier cas, le rapport entre la quantité de liquide provenant du point 12 et la quantité provenant d'une éventuelle fuite du dispositif de purge est avantageusement inférieur à la précision requise pour la mesure des paramètres physico-chimiques dans la chambre de mesure.

De préférence, le dispositif de purge 50 est disposé entre la chambre de mesure et le point de prélèvement ayant la plus faible profondeur. Ainsi, si le point de prélèvement de plus faible profondeur est à proximité du dispositif de purge et si ce dernier présente une fuite, le liquide issu de cette fuite ne fausse pas ou fausse peu la mesure puisque le liquide introduit dans le circuit hydraulique par cette fuite présente a priori les mêmes propriétés physico-chimiques que le liquide prélevé.

Pour préserver dans le temps l'étanchéité du dispositif de purge 50, le corps tubulaire 51 et le clapet 52 sont avantageusement réalisés en un matériau à surface biocide, par exemple en alliage cuivreux anti-bio salissure.

L'immersion du circuit hydraulique apporte de nombreux avantages:
- d'un point de vue énergétique le seul travail hydraulique consiste à faire circuler le liquide dans le circuit hydraulique en s'opposant aux pertes de charges linéaires et singulières, lesquelles sont très faibles en raison de la disposition en ligne des éléments du circuit hydraulique; il n'y a pas de travail d'élévation gravitaire de liquide à effectuer;
- l'étanchéité à un milieu liquide est plus facile à réaliser que l'étanchéité à l'air; de plus, les microfuites éventuelles au niveau du dispositif de purge automatique n'ont pas de conséquence sur le fonctionnement hydraulique du système;
- le dispositif de purge immergé est de structure très simple, ce qui ajoute à la fiabilité du système de mesure;
- le système ne requiert pas de circuit d'amorçage.

Bien que le système de l'invention fonctionne préférablement en totale immersion, il peut également fonctionner en partie émergé. Le dispositif de purge est alors changé par un dispositif de purge aérien, tel qu'une pompe à vide étanche à l'air.

A noter que le dispositif de purge immergé illustré aux figures 1 à 6 peut être remplacé par un dispositif plus classique, tel qu'une électrovanne commandée en ouverture entre deux cycles d'aspiration des pompes. De manière générale, le dispositif de purge est disposé en un point haut du circuit hydraulique sur une ouverture supérieure d'une conduite reliant la chambre de mesure à l'une des pompes.

## Revendications

1. Système de mesure ou de surveillance de la qualité d'un milieu liquide, comprenant des premier et second points de prélèvement d'échantillon (11, 12) dans le milieu liquide et un circuit hydraulique dans lequel circulent les échantillons prélevés à partir desdits premier et second points de prélèvement d'échantillon, lequel circuit hydraulique comprend :
- une chambre de mesure (20) dotée d'au moins un capteur de mesure (23) apte à mesurer une grandeur caractéristique dudit milieu liquide, tel qu'un paramètre physico-chimique ou biologique du milieu liquide, ladite chambre de mesure présentant une première ouverture (21) en communication fluidique avec le premier point de prélèvement d'échantillon et une deuxième ouverture (22),
- une première pompe (30) aspirante présentant une entrée d'aspiration (31) reliée à la seconde ouverture de la chambre de mesure et une sortie de refoulement (32) en communication fluidique avec le milieu liquide, ladite première pompe aspirante étant apte en configuration de fonctionnement à faire circuler dans le circuit hydraulique un échantillon prélevé à partir du premier point de prélèvement d'échantillon;
**caractérisé en ce que** le circuit hydraulique (C) comporte en outre
- une deuxième pompe (40) aspirante présentant une entrée d'aspiration (41) reliée à la première ouverture de la chambre de mesure et une sortie de refoulement (42) en communication fluidique avec le milieu liquide, ladite deuxième pompe aspirante étant apte en configuration de fonctionnement à faire circuler dans le circuit hydraulique un échantillon prélevé à partir du deuxième point de prélèvement d'échantillon;
- au moins une conduite (60) pour raccorder la première ouverture de la chambre de mesure à l'entrée d'aspiration de la deuxième pompe aspirante ou raccorder la deuxième ouverture de la chambre de mesure à l'entrée d'aspiration de la première pompe aspirante, ladite conduite étant munie d'un dispositif de purge (50) pour évacuer les gaz présents dans le circuit hydraulique,
et **en ce que** la sortie de refoulement (32) de la première pompe aspirante est raccordée au deuxième point de prélèvement d'échantillon (12) et la sortie de refoulement (42) de la deuxième pompe aspirante est raccordée au premier point de prélèvement d'échantillon (11), lesdites première et deuxième pompes aspirantes étant, à l'arrêt, aptes à autoriser le passage de liquide de leur sortie de refoulement vers leur entrée d'aspiration.

2. Système selon la revendication 1, **caractérisé en ce que** les première et seconde pompes (30,40) aspirantes sont adaptées à une immersion et sont, en configuration de fonctionnement, immergées dans le milieu liquide.

3. Système selon la revendication 1 ou 2, **caractérisé en ce que** le dispositif de purge (50) est adapté à une immersion et est immergé dans le milieu liquide.

4. Système selon la revendication 3, **caractérisé en ce que** le dispositif de purge (50) comprend un système de clapet pour évacuer les gaz présents dans le circuit hydraulique.

5. Système selon la revendication 4, **caractérisé en ce que** le dispositif de purge (50) comprend:
- un corps tubulaire (51) sensiblement vertical monté par son extrémité (512) ouverte inférieure sur une ouverture supérieure de la conduite (60) entre la chambre de mesure et la première ou deuxième pompe aspirante, et
- un clapet (52) cylindrique apte à coulisser dans le passage interne (510) du corps tubulaire et à coopérer avec une portion inférieure (514) du corps tubulaire, formant siège, pour obturer l'extrémité (512) ouverte inférieure dudit corps tubulaire.

6. Système selon la revendication 5, **caractérisé en ce que** la paroi intérieure de la portion inférieure (514) du corps tubulaire comprend une portion tronconique (514a) formant le siège du clapet.

7. Système selon la revendication 6, **caractérisé en ce que** le clapet (52) comporte une portion inférieure (521) dont la paroi extérieure comprend une portion tronconique coopérant avec la portion tronconique (514a) de la paroi intérieure du corps tubulaire pour obturer l'extrémité (512) ouverte inférieure du corps tubulaire.

8. Système selon l'une quelconque des revendications précédentes, **caractérisé en ce que** le circuit hydraulique comprend un moyen injecteur ou générateur de produit biocide (24) disposé entre desdits premier et second points de prélèvement d'échantillon (11, 12).

9. Système selon la revendication 8, **caractérisé en ce que** ledit moyen générateur de produit biocide (24) est monté sur la chambre de mesure.

10. Système selon l'une quelconque des revendications précédentes, **caractérisé en ce que** lesdites première et seconde pompes (30,40) aspirantes sont des pompes centrifuges à rouet.

## Patentansprüche

1. System zum Messen oder Überwachen der Qualität eines flüssigen Mediums, das einen ersten und einen zweiten Probenabtastpunkt (11, 12) in dem flüssigen Medium und einen Hydraulikkreis, in dem die abgetasteten Proben ausgehend von dem ersten und dem zweiten Probenabtastpunkt zirkulieren, umfasst, wobei der Hydraulikkreis Folgendes umfasst:
- eine Messkammer (20), die mit wenigstens einem Messsensor (23) versehen ist, der eine Kenngröße des flüssigen Mediums wie etwa einen physikalischchemischen oder einen biologischen Parameter des flüssigen Mediums messen kann, wobei die Messkammer eine erste Öffnung (21) in Fluidkommunikation mit dem ersten Probenabtastpunkt und eine zweite Öffnung (22) aufweist,
- eine erste Saugpumpe (30), die einen Saugeingang (31), der mit der zweiten Öffnung der Messkammer verbunden ist, und einen Förderausgang (32) in Fluidkommunikation mit dem flüssigen Medium aufweist, wobei die erste Saugpumpe eine Betriebskonfiguration haben kann, um in dem Hydraulikkreis eine abgetastete Probe ausgehend von dem dritten Probenabtastpunkt zirkulieren zu lassen;
**dadurch gekennzeichnet, dass** der Hydraulkkreis (C) außerdem Folgendes umfasst:
- eine zweite Saugpumpe (40), die einen Saugeingang (41), der mit der ersten Öffnung der Messkammer verbunden ist, und einen Förderausgang (42) in Fluidkommunikation mit dem flüssigen Medium aufweist, wobei die zweite Saugpumpe eine Betriebskonfiguration haben kann, um eine abgetastete Probe in dem Hydraulikkreis ausgehend von dem zweiten Probenabtastpunkt zirkulieren zu lassen;
- wenigstens eine Leitung (60), um die erste Öffnung der Messkammer mit dem Saugeingang der zweiten Saugpumpe zu verbinden oder um die zweite Öffnung der Messkammer mit dem Saugeingang der ersten Saugpumpe zu verbinden, wobei die Leitung mit einer Entleerungsvorrichtung (50) versehen ist, um die in dem Hydraulikkreis vorhandenen Gase abzuführen,
und dass der Förderausgang (32) der ersten Saugpumpe mit dem zweiten Probenabtastpunkt (12) verbunden ist und der Förderausgang (42) der zweiten Saugpumpe mit dem ersten Probenabtastpunkt (11) verbunden ist, wobei die erste und die zweite Saugpumpe im Ruhezustand den Durchgang von Flüssigkeit von ihrem Förderausgang zu ihrem Saugeingang zulassen können.

2. System nach Anspruch 1, **dadurch gekennzeichnet, dass** die erste und die zweite Saugpumpe (30, 40) eingetaucht werden können und in der Betriebskonfiguration in das flüssige Medium eingetaucht sind.

3. System nach Anspruch 1 oder 2, **dadurch gekennzeichnet, dass** die Entleerungsvorrichtung (50) für ein Eintauchen geeignet ist und in das flüssige Medium eingetaucht ist.

4. System nach Anspruch 3, **dadurch gekennzeichnet, dass** die Entleerungsvorrichtung (50) ein Ventilsystem umfasst, um die in dem Hydraulikkreis vorhandenen Gase abzuführen.

5. System nach Anspruch 4, **dadurch gekennzeichnet, dass** die Entleerungsvorrichtung (50) Folgendes umfasst:
- einen im Wesentlichen vertikalen rohrförmigen Körper (51), der mit seinem unteren offenen Ende (512) auf einer oberen Öffnung der Leitung (60) zwischen der Messkammer und der ersten oder der zweiten Saugpumpe montiert ist und
- ein zylindrisches Ventil (52), das in dem inneren Durchlass (510) des rohrförmigen Körpers gleiten kann und mit einem unteren Abschnitt (514) des rohrförmigen Körpers, der einen Sitz bildet, zusammenwirken kann, um das untere offene Ende (512) des rohrförmigen Körpers zu verschließen.

6. System nach Anspruch 5, **dadurch gekennzeichnet, dass** die Innenwand des unteren Abschnitts (514) des rohrförmigen Körpers einen kegelstumpfförmigen Abschnitt (514a) aufweist, der den Sitz des Ventils bildet.

7. System nach Anspruch 6, **dadurch gekennzeichnet, dass** das Ventil (52) einen unteren Abschnitt (521) aufweist, dessen Außenwand einen kegelstumpfförmigen Abschnitt aufweist, der mit dem kegelstumpfförmigen Abschnitt (514a) der Innenwand des rohrförmigen Körpers zusammenwirkt, um das untere offene Ende (512) des rohrförmigen Körpers zu verschließen.

8. System nach einem der vorhergehenden Ansprüche, **dadurch gekennzeichnet, dass** der Hydraulikkreis ein Einspritz- oder Generatormittel (24) für ein Biozidprodukt umfasst, das zwischen dem ersten und dem zweiten Probenabtastpunkt (11, 12) angeordnet ist.

9. System nach Anspruch 8, **dadurch gekennzeichnet, dass** das Biozidprodukt-Generatormittel (24) an der Messkammer montiert ist.

10. System nach einem der vorhergehenden Ansprüche, **dadurch gekennzeichnet, dass** die erste und die zweite Saugpumpe (30, 40) Flügelrad-Zentrifugenpumpen sind.

## Claims

1. System for measuring or monitoring the quality of a liquid medium comprising first and second points for taking off a sample from the liquid medium and a hydraulic circuit in which the samples (11, 12) taken from said first and second sample-takeoff points circulate, said hydraulic circuit comprising:
- a measuring chamber (20) provided with at least one measuring sensor (23) able to measure a quantity characteristic of the liquid medium, such as a physicochemical or biological parameter of the liquid medium, said measuring chamber having a first opening (21) in fluid communication with the first sample-takeoff point and a second opening (22), and
- a first suction pump (30) having a suction inlet (31) connected to the second opening of the measuring chamber and a discharge opening (32) in fluid communication with the liquid medium, the first suction pump being able, in operating configuration, to circulate in the hydraulic circuit a sample taken from the first sample-takeoff point;
**characterised in that** the hydraulic circuit (C) also comprises
- a second suction pump (40) having a suction inlet (41) connected to the first opening of the measuring chamber and a discharge outlet (42) in fluid communication with the liquid medium, said second suction pump being able, in the operating configuration, to circulate in the hydraulic circuit a sample taken from the second sample-takeoff point;
- at least one conduit (60) for connecting the first opening of the measuring chamber to the suction inlet of the second suction pump or connecting the second opening of the measuring chamber to the suction inlet of the first suction pump, said conduit being provided with a purge device (50) in order to discharge the gases present in the hydraulic circuit,
and **in that** the discharge outlet (32) of the first suction pump is connected to the second sample-takeoff point (12) and the discharge outlet (42) of the second suction pump is connected to the first sample-takeoff point (11), said first and second suction pumps being, at rest, able to allow the passage of liquid from their discharge outlet to their suction inlet.

2. System according to claim 1, **characterised in that** the first and second suction pumps (30, 40) are suitable for immersion and are, in the operating configuration, immersed in the liquid medium.

3. System according to claim 1 or 2, **characterised in that** the purge device (50) is suitable for immersion and is immersed in the liquid medium.

4. System according to claim 3, **characterised in that** the purge device (50) comprises a valve system for discharging the gases present in the hydraulic circuit.

5. System according to claim 4, **characterised in that** the purge device (50) comprises:
- a substantially vertical tubular body (51) mounted by its bottom open end (512) on a top opening of the conduit (60) between the measuring chamber and the first or second suction pump, and
- a cylindrical valve (52) able to slide in the internal passage (510) in the tubular body and to cooperate with a bottom portion (514) of the tubular body, forming a seat, in order to close off the bottom open end (512) of said tubular body.

6. System according to claim 5, **characterised in that** the internal wall of the bottom portion (514) of the tubular body comprises a frustoconical portion (514a) forming the seat of the valve.

7. System according to claim 6, **characterised in that** the valve (52) comprises a bottom portion (521) the external wall of which comprises a frustoconical portion cooperating with the frustoconical portion (514a) of the internal wall of the tubular body in order to close off the bottom open end (512) of the tubular body.

8. System according to any one of the preceding claims, **characterised in that** the hydraulic circuit comprises a means of injecting or generating a biocidal product (24) placed between said first and second sample-takeoff points (11, 12).

9. System according to claim 8, **characterised in that** said biocidal product generating means (24) is mounted on the measuring chamber.

10. System according to any one of the preceding claims, **characterised in that** said first and second suction pumps (30, 40) are centrifugal impeller pumps.
